# FASCICULE DE BREVET EUROPEEN

(11) **EP 2 814 564 B1**
(45) Date de publication et mention de la délivrance du brevet: **25.05.2016**
(21) Numéro de dépôt: 13704480.6
(22) Date de dépôt: 17.01.2013
(51) Int. Cl.: A61N 1/16, H04B 1/38, H01Q 1/24

(54) **DISPOSITIF D'ENVELOPPE MULTICOUCHE D'ATTENUATION DES ONDES ELECTROMAGNETIQUES**
MEHRSCHICHTIGES GEHÄUSE VORRICHTUNG ZUR ABSCHWÄCHUNG ELEKTROMAGNETISCHER WELLEN
MULTILAYER CASING DEVICE FOR ATTENUATING ELECTROMAGNETIC WAVES

(30) Priorité: 01.02.2012 FR 1250943
(43) Date de publication de la demande: 24.12.2014
(73) Titulaire: Duthilleul, Pascal, 34190 Ganges (FR)
(72) Inventeur: DUTHILLEUL, Pascal, 34190 Ganges (FR)
(74) Mandataire: Cornuejols, Georges
(86) Numéro de dépôt international: PCT/FR2013/050107
(87) Numéro de publication internationale: WO 2013/114019

(56) Documents cités:
- EP-A1- 0 915 572
- EP-A1- 1 174 162
- EP-A1- 1 981 324
- WO-A1-2008/044414
- JP-A- 2001 217 590

## Description

La présente description entre dans le domaine de l'électromagnétisme, plus particulièrement dans la protection à l'encontre des ondes électromagnétiques.

L'invention concerne un dispositif d'enveloppe multicouche d'atténuation des ondes électromagnétiques, tel que défini dans la revendication 1.

Un tel dispositif trouvera une application préférentielle, mais aucunement limitative, dans l'atténuation du rayonnement émis par des terminaux mobiles, notamment de type téléphone cellulaire. Pour ce faire, le dispositif d'enveloppe multicouche selon la présente demande se présente sous la forme d'un matériau souple ou semi-rigide et peut être conformé en pochette, à l'intérieure de laquelle est placé ledit terminal, dès lors entouré par cette enveloppe.

A l'heure actuelle, de nombreuses technologies de la vie courante utilisent les fréquences radio, pour l'émission et la réception d'informations. Ces technologies génèrent alors un champ électromagnétique qui, à proximité d'être vivant, sont susceptibles d'interférer avec le fonctionnement biologique, notamment au niveau du système nerveux.

Il a été fréquemment observé chez des personnes exposées de façon récurrente à un champ électromagnétique plusieurs symptômes, à savoir maux de tête, accroissement du stress, nervosité et irritabilité, troubles du sommeil, fatigue accrue, mais aussi un affaiblissement des défenses immunitaires, des trouble de la concentration et de la mémoire, des troubles neuro-endocrino-immunitaires, et certains champs seraient à la source d'une augmentation des risques de cancer et de fausses couches.

Dans ce contexte, les téléphones cellulaires dits « mobiles » ou « portables » sont des transmetteurs de radiofréquences, opérant à des fréquences situées entre 450 et 2 700 MHz, pour une émission maximale d'une puissance se situant entre 0,1 et 2 Watts. Si de tels transmetteurs sont de faible énergie, les durées d'exposition avec leur utilisateur dépassent plusieurs heures par jour, mais l'influence peut être accrue en raison leur proximité d'utilisation, au contact de l'oreille lors de communication téléphonique ou bien porté sur soi, dans une poche, le reste du temps.

Dès lors, il est nécessaire de limiter l'exposition d'un utilisateur aux champs électromagnétiques générés par un terminal, sans pour autant dégrader les conditions de fonctionnement de ce dernier.

Une solution a imaginée au travers du document EP 1 981 324, décrivant une pochette pour téléphone cellulaire constituée d'un matériau créant une barrière pour protéger le téléphone de toute radiation ou émission. Ledit matériau est constitué de plusieurs couches formées d'un tissage de fils de trame et de chaine, dont les uns ou les autres sont constitués par un composé conducteur électriquement, notamment un métal sous forme de ruban comme du laiton, du nickel ou du cuivre. Toutefois, une telle coque bloque totalement le rayonnement électromagnétique, depuis ou vers le téléphone, qui peut alors rester allumé mais sera déconnecté du champ électromagnétique du réseau et des autres champs électromagnétiques.

Une autre solution consiste en une coque protectrice semi-rigide limitant le rayonnement des champs électromagnétiques issus d'un téléphone cellulaire. Telle que décrite au travers du document EP 0 915 572, ladite coque est réalisée en matériau élastomère, recouvert ou incluant un treillis ou maillage métallique, comme du polyester recouvert de cuivre et de nickel. Une telle coque n'apporte toutefois par entière satisfaction puisqu'elle recouvre qu'une partie du téléphone, ne limitant que le rayonnement électromagnétique au niveau de l'antenne d'émission et de réception des fréquences radio.

Une solution alternative consiste en un textile d'atténuation du rayonnement électromagnétique. Comme décrit dans le document WO 2002/05892, un tel textile est réalisé à base de fibres textiles et d'au moins un composé d'atténuation des champs électromagnétiques, contenus à hauteur de 5 à 15% au sein dudit textile. En particulier, ce dernier est constitué de fibres tissées avec lesquelles est tissé ou incorporé ledit composé d'atténuation, pouvant être, d'une part, métallique, comme de l'argent, de l'or, du platine ou du cuivre ou, d'autre part, un semi-conducteur, de type à structure cristalline contenant notamment du silicone, du sélénium, du germanium ou du bore. Ainsi, il est possible de fabriquer, en le tissant avec une fibre naturelle comme la laine ou le coton, le lin ou la soie, ou bien une fibre synthétique, comme le polyester ou l'acrylique, un textile en vue de réaliser un vêtement ou une partie de vêtement, comme une poche, au travers duquel le rayonnement électromagnétique est limité.

Toutefois, un tel textile présente l'inconvénient d'avoir une fabrication complexe et coûteuse, faisant intervenir des matériaux onéreux. De plus, le vêtement bloque le rayonnement, déconnectant le téléphone du réseau.

Une autre solution d'un domaine connexe est décrite au travers du document WO 2008/044414 et concerne un tablier de protection contre les champs électromagnétiques. En particulier, un tel tablier est constitué d'une structure multicouches conférant un pouvoir de blocage total des ondes électromagnétiques. En somme, la structure d'un tel tablier constitue un blindage que les ondes d'un champ électromagnétique ne peuvent traverser.

Un tel bouclier est préférentiellement destiné à servir de vêtement de protection, notamment pour les personnes portant un stimulateur cardiaque dont le fonctionnement est susceptible d'être affecté par un champ électromagnétique. Ce tablier permet ainsi de bloquer les ondes radio et les champs électromagnétiques, notamment provenant des plaques de cuisson à induction.

Plus précisément, se structure est recouverte en faces avant et arrière de couches constituées d'un matériau tissé de fibres synthétiques ou naturelles, notamment cités à titre d'exemple : en laine ou coton. On constate que ces couches textiles servent uniquement à conférer des couches de couverture à l'avant et à l'arrière du tablier, améliorant la finition pour que le tablier ressemble à un vêtement. Elles ne sont pas disposées pour jouer un rôle entre les autres couches.

De plus, ladite structure est constituée d'une couche d'absorption en cuivre et d'une couche de blocage ou "feuille de blindage" en aluminium. En outre, la couche de cuivre peut se présenter sous la forme d'une feuille, doublée de fer ou de nickel, d'une épaisseur comprise entre 10 et 35 microns. De préférence, l'épaisseur de 35 microns confère un blindage suffisant pour absorber le champ magnétique, tout en conservant une caractéristique flexible audit vêtement. Cette couche en cuivre joue donc un rôle d'absorption du magnétisme du champ électromagnétique.

Par ailleurs, la couche d'aluminium sert de réflecteur, utilisant le caractère imperméable au rayonnement électromagnétique de ce matériau. Elle coupe le champ électromagnétique qui n'a pas été absorbé par la couche de cuivre.

En somme, la couche de cuivre atténue le champ électromagnétique et la couche d'aluminium bloque le champ ainsi atténué. On notera que les couches de cuivre et d'aluminium sont séparées par des feuilles de carbone. Ces feuilles de carbones sont obtenues par polymérisation afin de constitue une structure multicouches flexible et uniformément compacte avec les couches de cuivre et d'aluminium. Il ne s'agit aucunement de couches de tissu.

Ainsi, la structure de ce dispositif permet de bloquer des champs électromagnétiques ayant des fréquences comprises entre 20 kHz à 2 Gigahertz (GHz), grâce à une couche d'aluminium pour bloquer totalement le champ électromagnétique. Toutefois, ce dispositif ne permet pas de simplement diminuer le champ électromagnétique, afin d'assurer le fonctionnement d'un appareil électronique, en particulier un téléphone cellulaire.

Le document WO 2008/044414 décrit un dispositif selon le préambule de la revendication 1.

La présente invention a pour but de pallier les inconvénients de l'état de la technique, en proposant un dispositif d'enveloppe multicouche d'atténuation des ondes électromagnétiques selon la revendication 1.

En particulier, ledit dispositif est constitué d'au moins un matériau textile et d'au moins un matériau métallique, caractérisé par le fait qu'il est constitué d'une superposition pourvue d'au moins d'une première couche en matériau métallique diamagnétique et constituée au moins en partie en cuivre, d' une seconde couche textile au moins en partie en laine ou en coton, d'une troisième couche constituée d'un matériau ferromagnétique, d'une quatrième couche textile au moins en partie en laine ou en coton, et qu'il est constitué d'une superposition complémentaire de couches identiques s'étendant symétriquement par rapport à ladite première couche de cuivre.

Une telle enveloppe confère au travers de sa superposition symétrique de couches, autour d'une âme métallique, une synergie permettant de canaliser le rayonnement électromagnétique, en particulier émis du téléphone, vers ladite âme. Cette dernière capte uniquement une partie de ce rayonnement pour le transformer, laissant filtrer un rayonnement atténué suffisant pour connecter ledit téléphone au réseau de communication. En particulier, la superposition selon l'invention permet de créer un effet Casimir permettant de modifier le rayonnement électromagnétique en le transformant en photons.

De plus, selon d'autres caractéristiques, ladite troisième couche peut être métallique et constituée au moins en partie en laine d'acier.

Selon un mode préférentiel de réalisation, ledit dispositif comprend au moins une couche périphérique de protection.

Selon un autre mode de réalisation, lesdites couches sont fixées ensembles cousues au moins au niveau de leur pourtour.

Ainsi, l'invention permet d'atténuer les ondes émises par le téléphone. Des tests ont montré une atténuation du niveau de DAS pour « Débit d'Absorption Spécifique » (ou SAR pour « Specific Absorption Rate ») en Watt par kilogramme (W/Kg) supérieure à 90 % lorsqu'un téléphone est équipé d'un dispositif selon l'invention.

D'autres mesures ont permis de mesurer une atténuation de la puissance d'émission en milliWatt (mW) supérieure à 70 % en moyenne pour un téléphone équipé dudit dispositif, soit en moyenne environ 10 mW d'absorption par le dispositif.

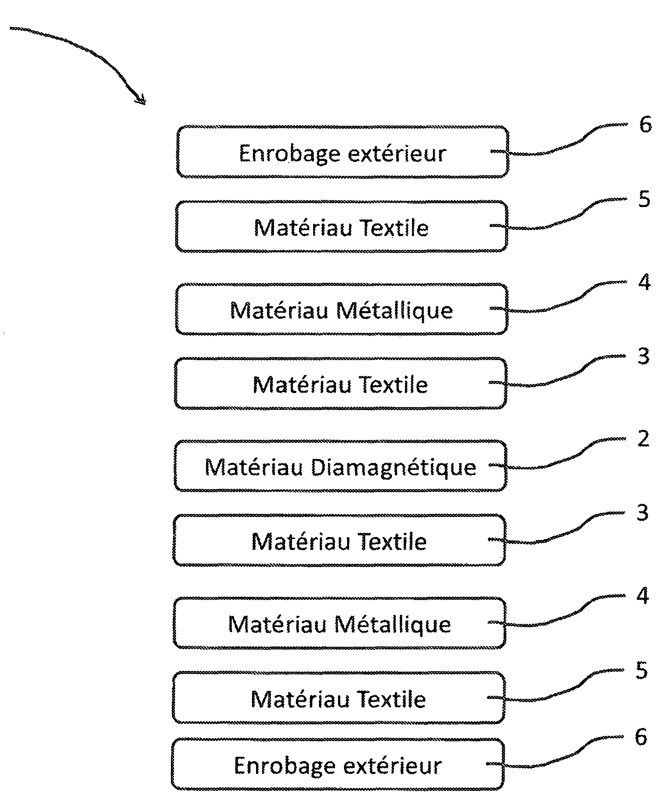

D'autres caractéristiques et avantages ressortiront de la description détaillée qui va suivre de modes de réalisation différents, en référence à la figure annexée représentant schématiquement la superposition de différentes couches selon un mode préférentiel de réalisation dudit dispositif d'enveloppe.

La présente invention selon la revendication 1 concerne un dispositif (1) d'enveloppe multicouche d'atténuation des ondes électromagnétiques.

Un tel dispositif (1) est constitué d'au moins un matériau textile et d'au moins un matériau métallique. Plus particulièrement, l'invention consiste en une synergie des actions de plusieurs couches en matériaux métalliques et textiles, placées de façon adjacentes.

Avantageusement, ledit dispositif (1) d'enveloppe est constitué d'une superposition pourvue d'au moins, successivement d'une première couche (2) en matériau diamagnétique, de préférence métallique, d'une seconde couche textile (3) présentant des caractéristiques de dipolarité électrostatique, d'une troisième couche (4) en matériau ferromagnétique, de préférence métallique, d'une quatrième couche (5) textile présentant des caractéristiques de dipolarité électrostatique.

Plus particulièrement, ladite première couche (2) diamagnétique est métallique et composée au moins en partie en cuivre, la seconde couche(3)textile au moins en partie en laine ou en coton, la troisième couche (4) ferromagnétique et métallique au moins en partie en laine d'acier, tandis que la quatrième couche textile (5) au moins en partie en laine ou en coton.

Selon le mode préférentiel de réalisation, ladite première couche (2) est constituée de cuivre à partir d'une feuille ou d'une plaque de cuivre ou en majorité constituée de cuivre. Cette feuille peut présenter différentes épaisseurs, notamment de l'ordre du millimètre.

Par ailleurs, les seconde (3) et quatrième (5) couches textiles peuvent être identiques, constituées d'un matériau tissé de fibres naturelles de laine, de coton ou d'un mélange des deux. Ces deux types de textile ont pour fonction d'offrir une couche isolante entre le cuivre et l'acier, ainsi qu' entre l'acier et l'extérieur. Ainsi, est créée une couche ayant des propriétés de dipolarité électrostatique, générant un champ différent lorsqu'elle est traversée par le rayonnement électromagnétique.

De plus, ladite troisième couche (4) peut être constituée d'un matelas fibreux de laine d'acier, d'une épaisseur pouvant varier, notamment de l'ordre du millimètre. En outre, la granulométrie de ladite laine d'acier peut varier, n° 0000, 0 ou 5. Cette couche (4) confère un effet Casimir, modifiant le champ électromagnétique pour le transformer au moins partiellement en photons. Par ailleurs, cet effet Casimir s'amplifie jusqu'à l'âme de l'enveloppe (1) constituée par la couche centrale de cuivre.

Selon le mode préférentiel de réalisation, représenté sur la figure, ledit dispositif (1) d'enveloppe peut être constitué d'une superposition complémentaire de couches identiques s'étendant symétriquement par rapport à ladite première couche (2) de cuivre. En somme, le cuivre sert d'âme centrale à l'enveloppe (1) et se succèdent de part et d'autre symétriquement des couches en matériau identiques, à savoir la seconde couche (3) en laine ou coton, la troisième couche (4) en laine d'acier et la quatrième couche (5) en laine ou coton.

On notera que si les matériaux sont identiques, l'épaisseur et la constitution des couches peuvent varier. En outre, ladite deuxième (3) ou troisième (4) couche d'un côté peut être constituée en laine tandis qu'elle est en coton de l'autre côté, ou inversement.

Avantageusement, cette combinaison particulière desdites couches (2,3,4,5) superposées génère un effet de canalisation du rayonnement électromagnétique vers la première couche (2) de cuivre, qui sert alors d'âme réceptrice au centre du matériau, le rayonnement converge alors vers la feuille de cuivre.

De plus, l'alternance de couches conductrices et isolantes procure un effet d'absorption par les couches ferromagnétiques (4) et de transformation du rayonnement électromagnétique en photons au niveau de la couche (2) de cuivre, cette dernière étant diamagnétique, elle est donc insensible au champs magnétiques.

Selon le même mode préférentiel de réalisation, ledit dispositif (1) peut comprendre au moins une couche périphérique (6) de protection. Cette dernière peut être constituée d'une couche d'enrobage (6) constituée d'un matériau inerte, sans influence lorsqu'il est traversé par un champ électromagnétique. Elle sert alors uniquement d'enrobage, en tant que couverture esthétique ou protectrice.

Selon un mode de réalisation, ladite couche périphérique 6 peut être constituée en cuir, naturel ou synthétique.

En vue de conformer le dispositif (1) d'enveloppe en fonction de son utilisation, lesdites couches (2,3,4,5,6) sont fixées ensembles, notamment cousues au moins au niveau de leur pourtour.

On notera que les couches (2.3,4,5,6) sont accolées les unes aux autres, sans espacement entre elles.

De plus, dans le cas préférentiel d'une utilisation en tant que coque ou pochette pour téléphone cellulaire, l'enveloppe (1) selon l'invention peut être découpée et cousue selon les dimensions dudit téléphone. En outre, des rabats ou des lanières de maintien peuvent être ajoutés pour assujettir le téléphone à sa coque.

Selon d'autres utilisations, ledit dispositif (1) pourra est être conformé de manière à s'adapter à d'autres terminaux, notamment ordinateur, tablette, micro-onde, etc.

Ainsi, le dispositif (1) d'enveloppe selon l'invention permet d'atténuer le rayonnement émis par un terminal, notamment un téléphone cellulaire, sans pour autant bloquer totalement le champ électromagnétique, assurant la connexion de l'appareil à son réseau de communication.

De plus, l'invention se veut simple à industrialiser, avec des matériaux peu onéreux, conférant des coûts de fabrication peu élevés.

L'invention est définie par la revendication 1.

## Revendications

1. Dispositif (1) d'enveloppe multicouche d'atténuation des ondes électromagnétiques, constitué d'au moins un matériau textile et d'au moins un matériau métallique, et constitué d'une superposition pourvue d'au moins, successivement depuis une face de l'enveloppe vers la face opposée de l'enveloppe, d'une première couche (2) en matériau métallique diamagnétique et constituée au moins en partie en cuivre, d'une seconde couche (3) textile au moins en partie en laine ou en coton, d'une troisième couche (4) constituée d'un matériau ferromagnétique, d'une quatrième (5) couche textile au moins en partie en laine ou en coton, et **caractérisé par le fait qu'**il est constitué d'une superposition complémentaire de couches (3, 4, 5) identiques s'étendant symétriquement par rapport à ladite première couche (2) de cuivre.

2. Dispositif (1) d'enveloppe selon la revendication 1, **caractérisé par le fait que** ladite troisième couche (4) est métallique et constituée au moins en partie en laine d'acier.

3. Dispositif (1) d'enveloppe selon l'une quelconque des revendications précédentes, **caractérisé par le fait qu'**il comprend au moins une couche périphérique de protection (6).

4. Dispositif (1) d'enveloppe selon la revendication 3, **caractérisé par le fait que** lesdites couches (2, 3, 4, 5, 6) sont fixées ensembles cousues au moins au niveau de leur pourtour.

## Patentansprüche

1. Mehrschichtige Hüllenvorrichtung (1) zum Abschwächen elektromagnetischer Wellen, die durch zumindest ein Textilmaterial und zumindest ein metallisches Material gebildet wird, und durch eine Überlagerung gebildet wird, die nacheinander von einer Seite der Hülle zur gegenüber liegenden Seite der Hülle mit zumindest einer ersten Schicht (2) aus einem diamagnetischen metallischen Material versehen ist, und zumindest teilweise aus Kupfer gebildet wird, mit einer zweiten textilen Schicht (3), zumindest teilweise aus Wolle oder aus Baumwolle, mit einer dritten Schicht (4), die aus einem ferromagnetischen Material gebildet wird, mit einer vierten textilen Schicht (5) zumindest teilweise aus Wolle oder aus Baumwolle, und **dadurch gekennzeichnet, dass** sie aus einer ergänzenden Überlagerung von identischen Schichten (3, 4, 5) gebildet wird, die sich im Verhältnis zur besagten ersten Schicht (2) aus Kupfer symmetrisch erstrecken.

2. Hüllenvorrichtung (1) nach Anspruch 1, **dadurch gekennzeichnet, dass** die besagte dritte Schicht (4) metallisch ist, und zumindest teilweise aus Stahlwolle gebildet wird.

3. Hüllenvorrichtung (1) nach irgendeinem der vorherigen Ansprüche, **dadurch gekennzeichnet, dass** sie zumindest eine umlaufende Schutzschicht (6) umfasst.

4. Hüllenvorrichtung (1) nach Anspruch 3, **dadurch gekennzeichnet, dass** die besagten Schichten (2, 3, 4, 5, 6) zumindest im Bereich ihrer Umrandung durch Vernähen miteinander verbunden sind.

## Claims

1. Multilayer casing device (1) for attenuating electromagnetic waves made of at least one textile material and of at least one metallic material, **characterized in that** it consists in a superimposition provided with at least, successively from one surface of the casing to the opposite surface of the casing, a first layer (2) made of metallic diamagnetic material consisting at least in part of copper, a textile second layer (3) at least partly in wool or cotton, a third layer (4) made of a ferromagnetic material, a textile fourth layer (5) at least partly in wool or cotton, and **in that** it consists in an additional superimposition of identical layers (3, 4, 5) extending symmetrically in relation to said first copper layer (2).

2. Casing device (1) according to claim 1, **characterized in that** said third layer (4) is metallic and made at least in part of steel wool.

3. Casing device (1) according to any one of the preceding claims, **characterized in that** it comprises at least one peripheral protection layer (6).

4. Casing device (1) according to claim 3, **characterized in that** said layers (2, 3, 4, 5, 6) are fastened together, stitched at least at their perimeter.
